# EUROPÄISCHE PATENTANMELDUNG

(11) **EP 1 746 479 A1**
(43) Veröffentlichungstag der Anmeldung: **24.01.2007**
(21) Anmeldenummer: 05015965.6
(22) Anmeldetag: 22.07.2005
(51) Int. Cl.: G05D 16/06, A61M 5/152, A61M 39/22

(54) **Mechanisch betriebene Flüssigkeitspumpe**

(71) Anmelder: RoweMed AG - Medical 4 Life, 19370 Parchim (DE)
(72) Erfinder: Wex, Roland, 34212 Melsungen (DE); Hansmann, Harald, Prof. Dr.-Ing, 23966 Wismar (DE); Kenzler, Christian, 16928 Pritzwalk (DE)
(74) Vertreter: Quermann, Helmut

(57) **Zusammenfassung**

Die Erfindung schlägt eine mechanisch betriebene Flüssigkeitspumpe (1), insbesondere für medizinische oder ernährungsphysiologische Flüssigkeiten, sowie Flüssigkeiten im biologischen und Laborbereich vor. Bei dieser Pumpe sind folgende Funktionsteile innerhalb eines Gehäuses (2) der Pumpe gelagert:
ein verschließbarer Zugang (21) zum Zuführen der Flüssigkeit,
- ein aufweitbares elastisches Element (16) zum Speichern und Abgeben der über den verschließbaren Zugang zugeführten Flüssigkeit,
- eine Einrichtung (-, 32) zum weitgehenden Konstanthalten und/oder Begrenzen des von dem elastischen Element ausgegebenen Flüssigkeits-Volumenstroms,
- ein aus dem Gehäuse führender Auslass (48).

Bei einer solchen Pumpe befinden sich alle relevanten Funktionsteile in einer Funktionseinheit, so dass es nur noch erforderlich ist, die Pumpe mit Flüssigkeit zu befüllen und den Auslass anzuschließen. Insbesondere ist es nur erforderlich, einen Patienten an die Pumpe anzuschließen. Durch die Gestaltung der Pumpe ist deren Fehlbedienung ausgeschlossen. Die Pumpe baut klein und kompakt.

## Beschreibung

Die Erfindung betrifft eine mechanisch betriebene Flüssigkeitspumpe, insbesondere für medizinische oder ernährungsphysiologische Flüssigkeiten, sowie Flüssigkeiten im biologischen und Laborbereich.

Im Zusammenhang mit der Förderung medizinischer, ernährungsphysiologischer oder biologischer Flüssigkeiten oder Flüssigkeiten im Laborbereich werden die unterschiedlichsten Typen von Flüssigkeitspumpen verwendet. Es sind beispielsweise elektroenergetisch, elektrochemisch, gasförmig, mechanisch, elektromechanisch und mechanisch-physikalisch betriebene Pumpen bekannt. Viele dieser Pumpen können in aller Regel nur nach einer längeren Anlaufzeit eingesetzt werden und bieten dem Anwender und Verbraucher oftmals nur eine unzureichende Dosiergenauigkeit. Die bekannten Pumpensysteme sind im übrigen recht kostenaufwendig und unter ökologischem Gesichtspunkt wenig vorteilhaft. Sie können oftmals vom Anwender nicht tragbar benutzt werden.

Mechanisch betriebene Flüssigkeitspumpen zeichnen sich durch einen Antrieb aus, der in aller Regel recht einfach gestaltet sein kann. So sind unterschiedliche Ausführungsformen bekannt geworden, bei denen aufweitbare elastische Elemente zum Speichern und Abgeben der Flüssigkeit Verwendung finden.

Eine mechanisch betriebene Flüssigkeitspumpe, insbesondere für medizinische oder ernährungsphysiologische Flüssigkeiten ist aus der EP 0 944 405 B1 bekannt. Diese weist ein Gehäuse auf, das als Rohr ausgebildet ist. Innerhalb des Gehäuses ist ein aufweitbares elastisches Element zum Speichern und Abgeben der Flüssigkeit angeordnet, das als Schlauch ausgebildet ist. Dieser weist im Bereich seiner Enden Öffnungen auf. Mit dem Schlauch ist im Bereich der einen Öffnung eine Leitung zum Zuführen der Flüssigkeit zum Schlauch verbunden. Im Bereich der anderen Öffnung ist mit dem Schlauch eine weitere Leitung verbunden, die dem Abführen der Flüssigkeit aus dem Schlauch dient. Diese Leitung ist aus dem Gehäuse heraus geführt. Das dem Schlauch abgewandte Ende der Leitung ist mit einer Einrichtung zum Begrenzen des vom elastischen Element ausgegebenen Flüssigkeits-Volumenstroms versehen.

Aus der DE 100 58 121 A1 ist eine mechanisch betriebene Flüssigkeitspumpe, insbesondere für medizinische und ernährungsphysiologische Flüssigkeiten bekannt. Sie weist einen Beutel zur Aufnahme der Flüssigkeit und eine Ausgabeleitung für die Flüssigkeit auf. Der Beutel wird in ein Gehäuse der Pumpe eingelegt. Das Gehäuse weist ein erstes Gehäuseteil mit beidseitigen Scharnieren zur Aufnahme eines zweiten Gehäuseteils sowie eines Schließdeckels für das zweite Gehäuseteil auf. Beiden Gehäuseteilen ist eine Einrichtung zum Ausüben einer Druckkraft auf den Beutel zur Ausgabe der Flüssigkeit aus diesem zugeordnet. Diese Einrichtung weist ein elastisches Element zum Einwirken auf eine Beutelseite sowie das zweite Gehäuseteil zum Einwirken auf die gegenüberliegende Beutelseite auf. Die bei der Pumpe Verwendung findenden Beutel bzw. die Pumpe selbst können einmal verwendet oder mehrmals wieder verwendet werden. Je nach der gewählten Elastizität des elastischen Elements lassen sich bei der Pumpe unterschiedliche Flowraten und Entleerungszeiten erzielen. Das elastische Element ist im Rückstellfall so ausgelegt, dass die Flüssigkeit so lange aus dem Beutel ausgedrückt wird, bis das elastische Element an dem zweiten Gehäuseteil zum Einwirken auf die gegenüberliegende Beutelseite anliegt und in diesem Zustand der Beutel entleert ist.

In der EP 1 321 156 A1 ist ein Ventil, insbesondere für medizinische und ernährungsphysiologische Flüssigkeiten beschrieben. Dieses Ventil ermöglicht ein weitgehendes Konstanthalten des das Ventil durchsetzenden Flüssigkeitsstromes. Bestandteil des Ventils bildet ein Ventilkörper, der die Funktion eines aktiven Regelungselements besitzt, dessen Funktion von dem in einer Kammer des Ventils wirkenden Flüssigkeitsdruck abhängt. Steigt der Druck in der Flüssigkeitskammer, führt dies zu einer Stellbewegung des Ventilkörpers, die den Austritt der Flüssigkeit aus dem Eintrittskanal des Ventils drosselt, während eine Abnahme des Flüssigkeitsdruckes in der Kammer dazu führt, dass der Ventilkörper eine Bewegung vollführt, die die Drosselung der aus dem Eintrittskanal austretenden Flüssigkeit reduziert. In den Ausflussstutzen des Ventils ist ein Durchflussbegrenzer eingesetzt. Durch die Wahl verschiedener Durchflussbegrenzer, somit Ersetzen eines Durchflussbegrenzers durch einen anderen Durchflussbegrenzer, können verschiedene, konstante Flowraten des Ventils eingestellt werden.

In der EP 0 424 494 B1 ist eine Vorrichtung zur Ausgabe eines Fluids an einen Auslass beschrieben, wobei es sich bei dem Fluid insbesondere um eine medizinische Flüssigkeit handelt. Diese Flüssigkeit wird von einem aufweitbaren elastischen Element zum Speichern und Abgeben der Flüssigkeit an einen Schlauch abgegeben, der die Verbindung zu einer patientenseitigen Kanüle herstellt. Dem Schlauch ist eine Einrichtung zum Begrenzen des von dem elastischen Element ausgegebenen Flüssigkeits-Volumenstroms zugeordnet. Diese Leitung bildet die Hauptleitung. Vor der Begrenzungseinrichtung zweigt eine Nebenleitung ab, die hinter der Begrenzungseinrichtung wieder in die Hauptleitung mündet. Die Nebenleitung ist mit einer Bolusdosiseinrichtung versehen.

Allen vorstehend erörterten Einrichtungen ist gemeinsam, dass sie nur einige der insgesamt erforderlichen Funktionsteile für eine mechanisch betriebene Flüssigkeitspumpe aufweisen. Dies bedeutet, dass sie mit anderen erforderlichen Funktionsteilen kombiniert werden müssen. Diese Kombination erfolgt im Rahmen separater Einheiten, die demzufolge in der Anwendung miteinander verknüpft bzw. verbunden werden müssen. Dies bedeutet, insbesondere wenn medizinische oder ernährungsphysiologische Flüssigkeiten zu applizieren sind, einen hohen Handlingsaufwand und überdies ist eine erhebliche Gefahr einer Vertauschung oder eines falschen Anschlusses der Funktionsteile gegeben. Die Gefahr der Fehlbedienung eines aus einzelnen Funktionsteilen zusammengestellten Systems, das die Aufgabe einer mechanisch betriebenen Flüssigkeitspumpe besitzt, ist somit erheblich.

Aufgabe der vorliegenden Erfindung ist es, eine mechanisch betriebene Flüssigkeitspumpe, insbesondere für medizinische oder ernährungsphysiologische Flüssigkeiten, zu schaffen, bei der alle für die Funktion der Flüssigkeitspumpe erforderlichen Funktionsteile in einer Einheit untergebracht sind, die insbesondere kompakt gestaltet ist, wobei durch diese Anordnung der Funktionsteile sichergestellt sein soll, dass eine Fehlbedienung der Pumpe ausgeschlossen ist und demzufolge bei Anwendung der Pumpe im Bereich der Medizin, diese nur noch an den Patienten angeschlossen werden muss.

Gelöst wird die Aufgabe durch eine mechanisch betriebene Flüssigkeitspumpe, insbesondere für medizinische oder ernährungsphysiologische Flüssigkeiten, sowie Flüssigkeiten im biologischen und Laborbereich, mit folgenden innerhalb eines Gehäuses der Flüssigkeitspumpe gelagerten Funktionsteilen:
- einem verschließbaren Zugang zum Zuführen der Flüssigkeit,
- einem aufweitbaren elastischen Element zum Speichern und Abgeben der über den verschließbaren Zugang zugeführten Flüssigkeit,
- eine Einrichtung zum weitgehenden Konstanthalten und/oder Begrenzen des von dem elastischen Element ausgegebenen Flüssigkeits-Volumenstroms,
- einem aus dem Gehäuse führenden Auslass.

Erfindungsgemäß sind somit alle für die Funktion der Flüssigkeitspumpe wesentlichen Funktionsteile innerhalb des Gehäuses der Flüssigkeitspumpe angeordnet. Zur Inbetriebnahme der Flüssigkeitspumpe ist es demzufolge nur erforderlich, durch den verschließbaren Zugang die Flüssigkeit zuzuführen und den aus dem Gehäuse führenden Auslass mit dem Element zu verbinden, dass die aus der Pumpe ausgegebene Flüssigkeit weiter fördert, beispielsweise über einen LuerLock-Anschluss mit einem Schlauch, dessen dem Anschluss fernes Ende mit einem Katheter verbunden ist, der mit dem Patienten verbindbar ist. Der Auslass ist somit in diesem Fall zum Verbinden mit einer mit dem Patienten verbindbaren Einrichtung vorgesehen.

Die Bedienung der Flüssigkeitspumpe ist somit denkbar einfach. Fehlbedienungen sind ausgeschlossen, im Falle der Anwendung der Pumpe auf dem Gebiet der Medizin wird der Patient nur noch angeschlossen. Durch die erfindungsgemäße Gestaltung der Flüssigkeitspumpe mit den innerhalb des Gehäuses gelagerten Funktionsteilen besteht die Möglichkeit, die Pumpe klein und kompakt zu gestalten.

Es wird als besonders vorteilhaft angesehen, wenn die Funktionsteile kalibriert sind. Die Funktionsteile sind somit in ihrem physikalischen Verhalten aufeinander abgestimmt, so dass aus der Pumpe, unabhängig von dem Druckzustand im aufweitbaren elastischen Element, ein im wesentlichen konstanter Flüssigkeits-Volumenstrom ausgegeben wird.

Vorteilhaft ist das Gehäuse zumindest in einem Bereich, der den Zugang, die Einrichtung zum weitgehenden Konstanthalten und/oder Begrenzen des von dem elastischen Element ausgegebenen Flüssigkeits-Volumenstroms und den Auslass aufweist, insbesondere das Gehäuse insgesamt, nicht zerstörungsfrei demontierbar. Ist demzufolge das Gehäuse insgesamt bzw. in dem bezüglich der vorstehend genannten Funktionsteile befindlichen Bereich intakt, d.h. nicht zerstört, ist dies für den Nutzer der Pumpe eine Gewähr, dass an der Pumpe nicht manipuliert wurde.

Der Zugang und/oder der Abgang sind vorzugsweise als LuerLock-Ventil oder LuerLock-Anschluss ausgebildet. Es handelt sich somit um standardisierte Ventile bzw. Anschlüsse, die ein unkompliziertes Zuführen bzw. Abführen der Flüssigkeit gestatten.

Gemäß einer bevorzugten Ausgestaltung der Erfindung ist vorgesehen, dass das aufweitbare elastische Element als Ballon ausgebildet ist, der mit einer Öffnung versehen ist, wobei der Ballon abgedichtet in einem im Gehäuse gelagerten Kern gelagert ist und den Kern mindestens ein Kanal für den Zugang und den Abgang der Flüssigkeit in den Ballon bzw. aus dem Ballon durchsetzt. Es handelt sich somit um ein elastisches Element, das mit einer einzigen Öffnung versehen ist, im Gegensatz zu einem Schlauch mit zwei Öffnungen. Demzufolge dient diese Öffnung sowohl als Zugang für die Flüssigkeit als auch als Abgang für die Flüssigkeit. Der Ballon liegt vorzugsweise in relativ entspanntem Zustand am Kern an. Hierunter ist zu verstehen, dass der Ballon durchaus mit einer gewissen Vorspannung am Kern anliegen kann, da die vollständige Entleerung des Ballons anzustreben ist. Befestigt und abgedichtet wird der Ballon ausschließlich im Bereich seines die Öffnung aufweisenden Endes. Dies erfolgt vorzugsweise unmittelbar am Kern. Der Ballon kann grundsätzlich aus jedem Material gebildet sein, das die notwendige Elastizität zum Speichern und Abgeben der Flüssigkeit aufweist. Als bevorzugtes Material wird Silicon angesehen. Es ist insbesondere an ein Fassungsvermögen des Ballons von 10 ml bis 150 ml gedacht.

Bei der Flüssigkeitspumpe wird es als besonders wichtig angesehen, dass diese relativ flach ausgebildet ist, da sie in aller Regel von einem Patienten unmittelbar am Körper getragen wird. Um die Ausdehnung des Ballons vorzugsweise in Breitenerstreckung und weniger in seiner Höhe zu erreichen, ist vorgesehen, dass der Ballon in einer ersten Erstreckungsrichtung senkrecht zur Längsachse des Kerns relativ dicke Wandungsabschnitte und in einer zweiten Erstreckungsrichtung senkrecht zur Längsachse des Kerns und senkrecht zur ersten Erstreckungsrichtung relativ dünne Wandungsabschnitte aufweist. Das bedeutet, dass sich der Ballon stärker im Bereich der dünnen Wandungsabschnitte verformt, womit sich beim Dehnen des Elements eine im wesentlichen an die Form einer Ellipse angenäherte Querschnittsgestaltung ergibt. Der Ballon ist insbesondere durch Spritzgießen hergestellt.

Gemäß einer bevorzugten Gestaltung der Erfindung ist des weiteren vorgesehen, dass die Einrichtung zum Begrenzen des vom elastischen Element ausgegebenen Flüssigkeits-Volumenstromes als Ventil, insbesondere als Druckregelventil ausgebildet ist. Die Einrichtung zum weitgehenden Konstanthalten des vom elastischen Element ausgegebenen Flüssigkeits-Volumenstroms ist vorzugsweise als Durchflussbegrenzer, insbesondere als Glaskapillare oder Mäander-Chip ausgebildet. Innerhalb des Gehäuses, insbesondere stromaufwärts der Einrichtung zum weitgehenden Konstanthalten des vom elastischen Element ausgegebenen Flüssigkeits-Volumenstroms, ist vorzugsweise ein Filter-element für die Flüssigkeit angeordnet.

Das Gehäuse ist vorteilhaft mehrteilig gestaltet. Es weist insbesondere ein Mittelteil, ein Oberteil und ein Unterteil auf, wobei die Bezeichnung Oberteil und Unterteil vorstehend nur gewählt worden ist, um eine sprachliche Unterscheidung dieser Teile bezüglich des Mittelteils herbei zu führen. Da die Ausrichtung der Pumpe beliebig ist, kann das Oberteil das Unterteil und das Unterteil das Oberteil darstellen.

Aufgrund des Umstandes, dass das Gehäuse mehrteilig gestaltet ist, ist es möglich, den einzelnen Teilen des Gehäuses und den Trennebenen der einzelnen Gehäuseteile, beispielsweise dem Raum zwischen Mittelteil und Oberteil definierte Funktionen zuzuordnen: So weist das Gehäuse zweckmäßig eine mit dem Oberteil und dem Unterteil verbindbare, insbesondere unlösbar verclipsbare Haube, innerhalb der der Ballon und der Kern angeordnet ist, auf. Im Mittelteil und im Oberteil oder Unterteil, zwischen dem Mittelteil und dem Oberteil oder Unterteil, ist/sind zweckmäßig der Kern für den Ballon und/oder der Auslass und/oder das Filterelement und/oder die Einrichtung zum Begrenzen des vom elastischen Element ausgehenden Flüssigkeits-Volumenstroms gelagert. Im Mittelteil ist vorteilhaft die Einrichtung zum Begrenzen des vom elastischen Element ausgegebenen Flüssigkeits-Volumenstromes und/oder eine Boluseinrichtung gelagert. Im Oberteil oder Unterteil ist vorzugsweise der Zugang gelagert. Um die Funktionsteile, die durchaus auf der dem Mittelteil abgewandten Seite über das Oberteil und/oder das Unterteil hinausstehen können, abzudecken, ist ein Gehäuseoberteil zum Abdecken des Oberteils und ein Gehäuseunterteil zum Abdecken des Unterteils vorgesehen.

Die einzelnen Teile des Gehäuses bestehen vorzugsweise aus Kunststoff. Dies ermöglicht es, die Teile quasi beliebig zu formen und preisgünstig sowie bei geringem Gewicht herzustellen. Teile werden insbesondere durch Laserschweißen miteinander verbunden.

Eine besondere Funktion kommt bei dem Gehäuse dem Mittelteil zu. So ist vorgesehen, dass dieses nicht nur der Aufnahme von Funktionsteilen dient, sondern das Mittelteil auch die Strömungsverbindungen zwischen den Funktionsteilen aufweist, die insbesondere als in das Mittelteil eingeprägte Kanäle ausgebildet sind. Diese Kanäle werden abgedeckt durch Verbinden des Mittelteils mit dem Oberteil bzw. Unterteil, eine Flüssigkeitsdichtigkeit der Kanäle zwischen Mittelteil und Oberteil bzw. Unterteil wird insbesondere durch Laserschweißen erreicht.

Wie vorstehend ausgeführt, wird es bei der Pumpe als besonders wichtig angesehen, dass diese bzw. das Gehäuse relativ flach ausgebildet ist. Im Bereich des Ballons wird dies durch die besondere Querschnittsgestaltung des Ballons und die Ausbildung des Gehäuses als Haube erreicht. Auch der verbleibende Bereich des Gehäuses ist flach ausgebildet und nimmt die dort angeordneten Funktionsteile nebeneinander, auf unterschiedlichen Ebenen auf, einerseits im Bereich von Mittelteil und Oberteil, andererseits im Bereich von Mittelteil und Unterteil.

Die erfindungsgemäße mechanisch betriebene Flüssigkeitspumpe integriert somit alle Funktionsteile, die für ein einwandfreies Funktionieren der Flüssigkeitspumpe erforderlich sind und stellt sich somit vorteilhaft wie folgt dar:
- eine integrierte Lösung durch Wegfall eines externen Schlauchsystems zwischen Pumpe, Volumenstrombegrenzer und eventuell vorhandenem Bolusreservoir,
- ein integriertes Flüssigkeits-Zuführungssystem zu den einzelnen Komponenten durch eingeprägte Kanäle im Mittelteil,
- eine integrierte Pumpenlösung unter Verwendung eines Ballons, der mit einem Kern zusammenwirkt,
- einen integrierten Kapillar-/ Mäanderschutzfilter,
- einen integrierten Volumenstrombegrenzer in Kapillar- oder Mäanderchipform,
- eine eventuelle Boluslösung, die in Reihe oder als Bypass angeordnet sein kann,
- ein integriertes selbsttätiges Druckregelventil zur Flowraten-Konstantregelung.

Weitere Merkmale der Erfindung sind in den Unteransprüchen, der Beschreibung der Figuren sowie den Figuren selbst dargestellt, wobei bemerkt wird, dass alle Einzelmerkmale und alle Kombinationen von Einzelmerkmalen erfindungswesentlich sind.

In den Figuren ist die Erfindung anhand einer Ausführungsform beispielsweise dargestellt, ohne hierauf beschränkt zu sein. Es zeigt:
- Figur 1: eine Explosionsdarstellung der erfindungsgemäßen mechanisch betriebenen Flüssigkeitspumpe,
- Figur 2: einen vertikalen Längsmittelschnitt durch die in Figur 1 gezeigte Pumpe, bei am Kern anliegendem Ballon,
- Figur 3: ein Schnitt gemäß Figur 2, bei mit Flüssigkeit gefülltem Ballon,
- Figur 4: einen vertikalen Längsschnitt durch die in Figur 1 gezeigte Pumpe, in Abstand zur Längsmittelachse der Pumpe, im Bereich eines Ventils der Pumpe geschnitten,
- Figur 5: einen Schnitt, quer durch die in Figur 1 veranschaulichte Pumpe geschnitten, im Bereich des Ventils,
- Figur 6: einen horizontalen Längsmittelschnitt durch die in Figur 1 gezeigte Pumpe, bei am Kern anliegenden Ballon,
- Figur 7: einen Schnitt gemäß Figur 6, bei mit Flüssigkeit gefülltem Ballon,
- Figur 8: einen Schnitt durch die Pumpe, quer geschnitten im Bereich der Lagerung des Kernes,
- Figur 9: einen Schnitt durch die Pumpe, quer geschnitten im Bereich des nicht gelagerten Abschnitts des Kernes und des mit Flüssigkeit gefüllten Ballons,
- Figur 10: eine vergrößerte Schnittdarstellung von Kern, Ballon und Klemmring zum Verbinden von Ballon und Kern, bei am Kern anliegenden Ballon,
- Figur 11: einen Schnitt, quer zur Längserstreckung des Kerns geschnitten, durch den Kern und den Ballon, bei am Kern anliegenden Ballon,
- Figur 12: eine Schnittdarstellung gemäß Figur 11 für eine modifizierte Querschnittsgestaltung des Ballons,
- Figur 13: eine vergrößerte Schnittdarstellung des in Figur 4 gezeigten Ventils,
- Figur 14: ein Schaubild zur Verdeutlichung des Wirkprinzips der mechanisch betriebenen Flüssigkeitspumpe mit Angabe physikalischer Größen,
- Figur 15: ein Schaubild zur Verdeutlichung der grundsätzlichen Anordnung der bei der Pumpe verwendbaren Bolusschuss-Implementierung, bei Reihenschaltung,
- Figur 16: ein Schaubild gemäß Figur 15 für die Anordnung der Bolusschuss-Implementierung in Bypass-Schaltung und
- Figur 17: eine Schnittdarstellung einer bei der erfindungsgemäßen Pumpe Verwendung findenden Bolusschuss-Implementierung.

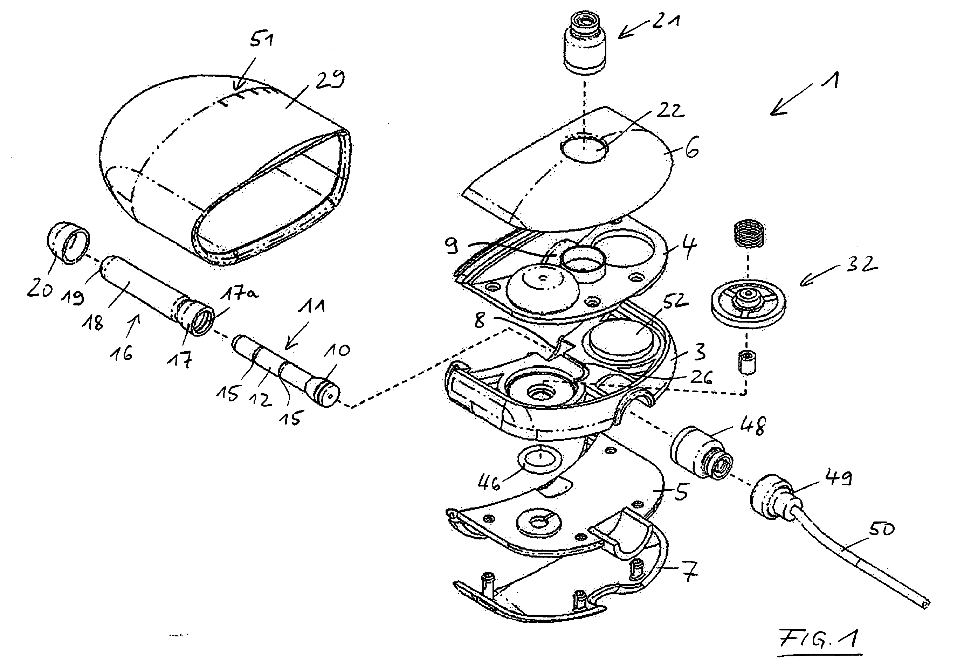

Die in Figur 1 veranschaulichte mechanisch betriebene Flüssigkeitspumpe 1 dient insbesondere der Applikation von medizinischen oder ernährungsphysiologischen Flüssigkeiten, beispielsweise der Applikation eines flüssigen Medikaments.

Die Pumpe 1 weist ein mehrteiliges Gehäuse 2 auf, das durch ein Mittelteil 3, mit diesem zusammenwirkenden Oberteil 4 und Unterteil 5, eine mit dem Oberteil zusammenwirkende Oberschale 6 und eine mit dem Unterteil 5 zusammenwirkende Unterschale 7 gebildet ist.

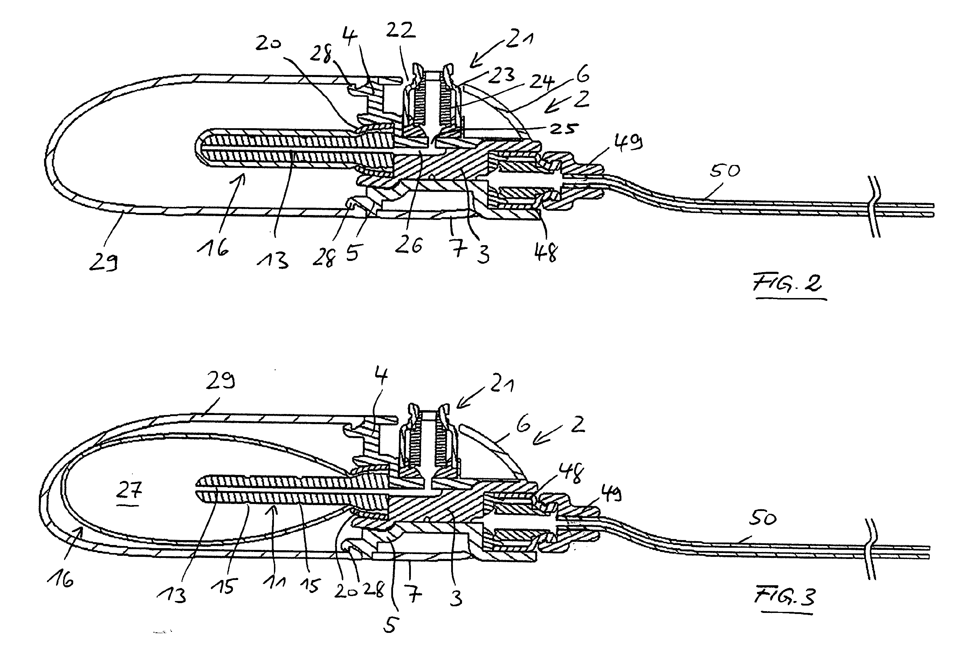

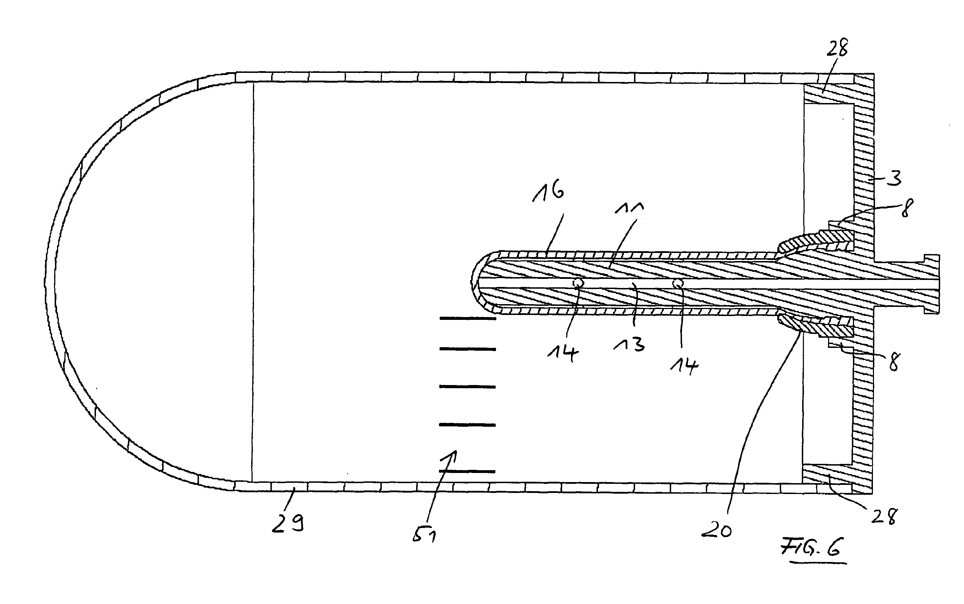

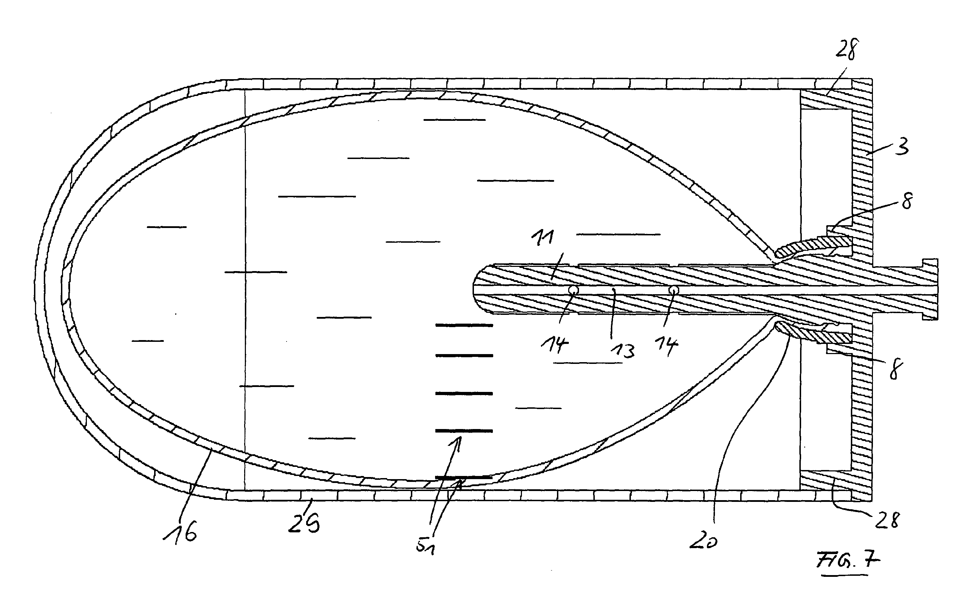

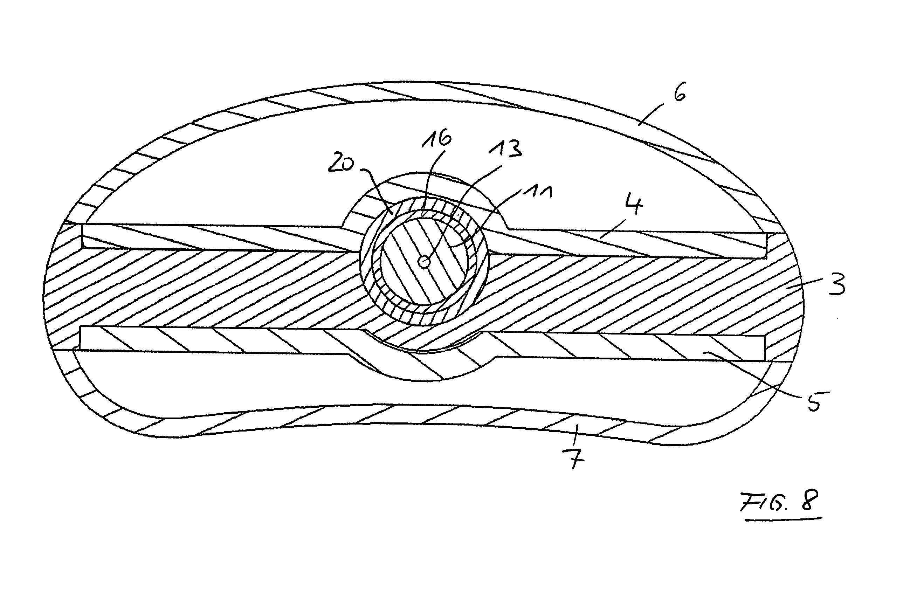

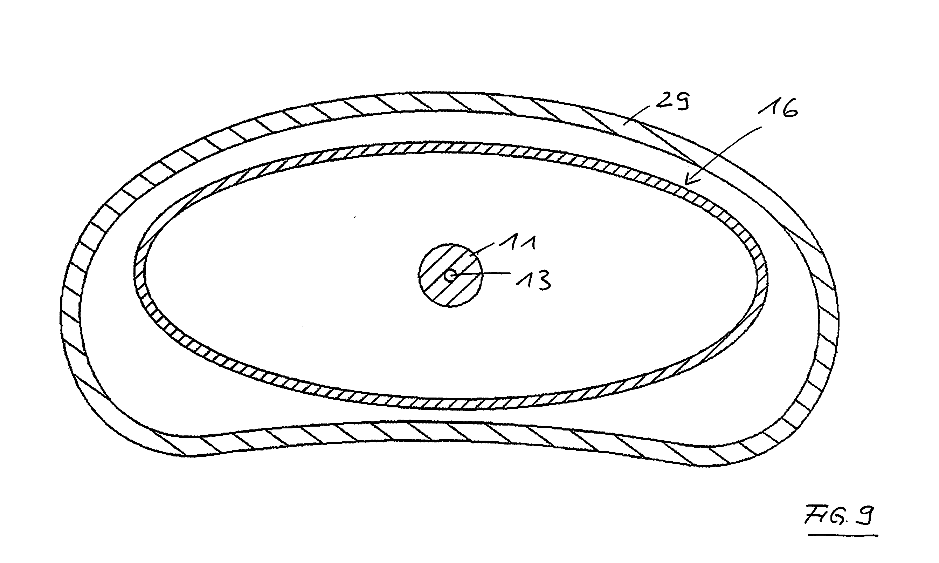

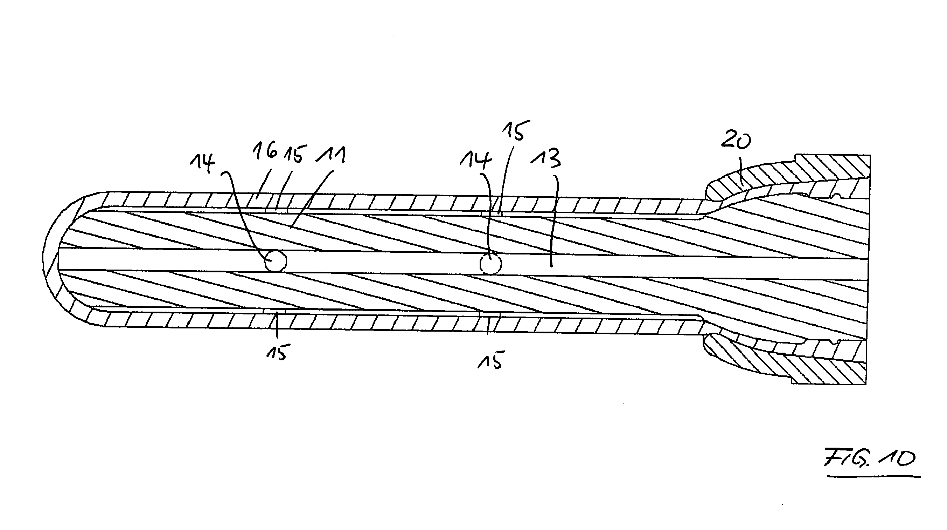

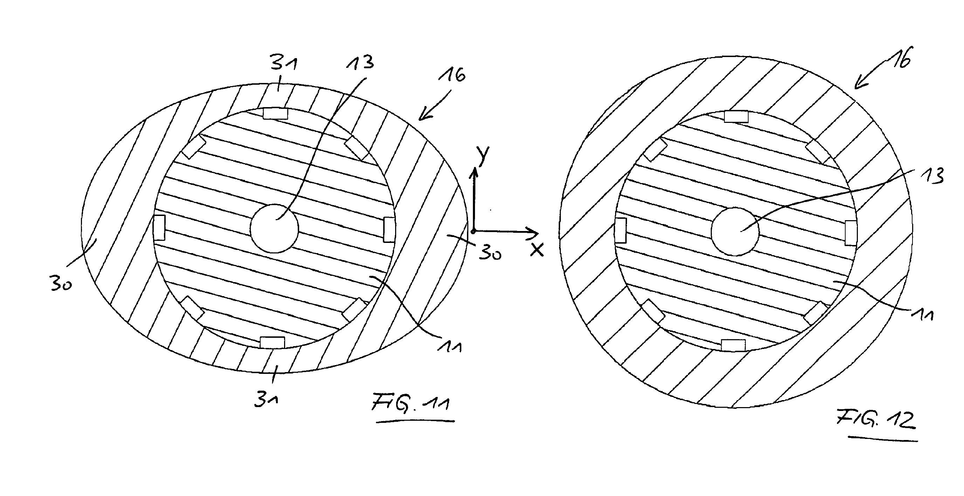

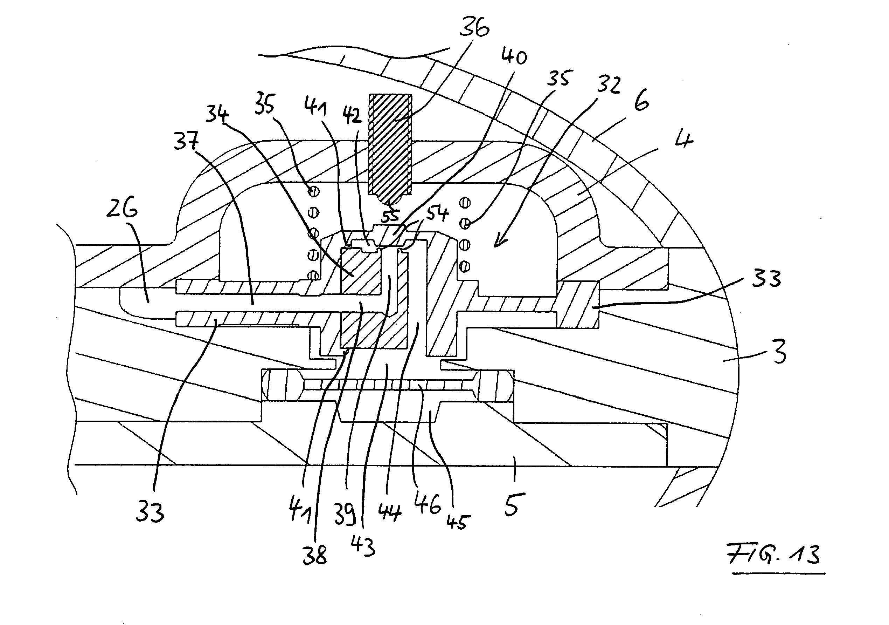

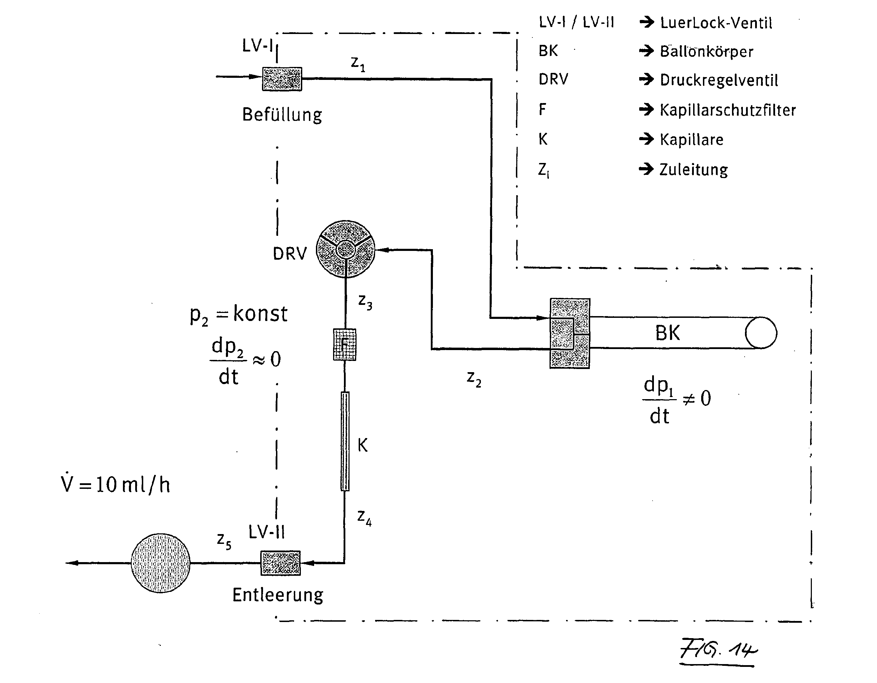

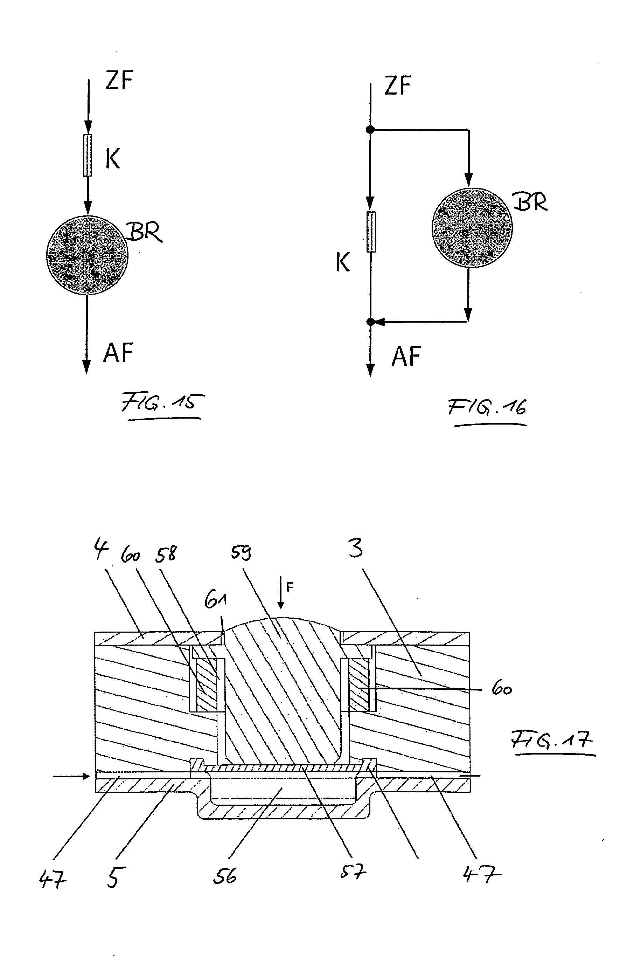

Das Mittelteil 3 ist auf seiner Oberseite mit einer zum freien Rand des Mittelteils 3 offenen, im Querschnitt halbkreisförmigen Ausnehmung 8 und das Oberteil 4 im entsprechenden Randbereich auf seiner Unterseite mit einer entsprechenden halbkreisförmigen Ausnehmung 9 versehen. Bei mit dem Mittelteil 3 verbundenem Oberteil 4 bilden die beiden Ausnehmungen 8 und 9 einen kreisförmigen Querschnitt zur Aufnahme eines konusförmig erweiterten Endbereichs 10 eines Kernes 11. Dieser weist, abgesehen von dessen Endbereich 10, einen konstanten Außendurchmesser auf. Dieser zylindrische Abschnitt des Kerns 11 ist mit der Bezugsziffer 12 bezeichnet. Den Kern 11 durchsetzt in dessen Längsmittelachse ein Kanal 13 (siehe Figur 2) von dem mehrere sich radial durch den Kern 11 erstreckende Kanäle 14 im Bereich des Abschnitts 12 abzweigen (Figur 6). Im Bereich des äußeren Umfangs des Kerns 10 münden die radialen Kanäle 14 in umlaufende Nuten 15 des Kerns 11.

Mit dem Kern 11 wirkt ein elastisches Element zusammen, dass als aus Silikon bestehender Ballon 16 ausgebildet ist. Dieser ist im Spritzgussverfahren hergestellt. Der Ballon weist entsprechend dem Endbereich 10 des Kerns 11 einen sich konisch erweiterten Endbereich 17 mit Öffnung 17a und einen der äußeren Form des Abschnitts 12 des Kerns 11 entsprechenden Abschnitt 18 auf, der in den, Ballon bedingt, geschlossenen Endbereich 19 mündet, der dem Endbereich 17 abgewandt ist.

Die Abmessungen von Kern 11 und Ballon 16 sind so bemessen, dass, wie es der Figur 2 zu entnehmen ist, der auf den Kern 11 aufgesteckte Ballon vollständig am Kern 11 anliegt, somit der Endbereich 17 des Ballons den Endbereich 10 des Kerns kontaktiert und der Abschnitt 18 des Ballons 16 den Abschnitt 12 des Kerns 11 kontaktiert, schließlich der Endbereich 19 des Ballons 16 am stirnseitigen freien Ende des Kerns 11 anliegt. Die Abmessungen des Ballons 16 bezüglich des Kerns 11 sind hierbei so gewählt, dass der Ballon 16 mit relativ geringer Vorspannung, somit in relativ entspanntem Zustand am Kern 11 anliegt.

Zum Befestigen des Ballons 16 im Bereich seines Endbereichs 17 am Kern 11 im Bereich dessen Endbereichs 10 ist ein Klemmring 20 vorgesehen, der außen auf den Ballon 16 im Bereich dessen Endbereichs 17 aufgesteckt wird. Das so geschaffene Gebilde wird mit dem Klemmring 20 in die Ausnehmung 8 des Mittelteils 3 eingelegt, anschließend das Oberteil 4 mit dem Mittelteil 3 verbunden, womit der Klemmring 20 und damit der Kern 11 sowie der Ballon 16 fest in den Ausnehmungen 8 und 9 von Mittelteil 3 und Oberteil 4 gehalten sind. Die Ausnehmungen 8 und 9 weisen eine sich vom jeweiligen freien Rand des Mittelteils 3 bzw. Oberteil 4 weg erweiternde konische Aufnahme für den Klemmring 20 auf, um einen sicheren Halt des Klemmrings 20 zu gewährleisten.

Das Mittelteil 3, das Oberteil 4 und das Unterteil 5 dienen der Aufnahme weiterer Funktionselemente der Pumpe 1:

Mit dem Oberteil 4 ist ein LuerLock-Ventil 21 verbunden, dass eine Öffnung 22 im Oberteil 4 durchsetzt und, wie es der nachfolgend Bezug genommenen Beschreibung der Figur 2 zu entnehmen ist, ein LuerLock-Ventilgehäuse 23 und einen LuerLock-Ventilkern 24 besitzt. Über einen Kanal 25 steht das LuerLock-Ventil 21 mit einem Kanal 26 in Verbindung, der zwischen dem Oberteil 4 und dem Mittelteil 3 gebildet ist und der mit dem den Kern 11 durchsetzenden Kanal 13 in Verbindung steht.

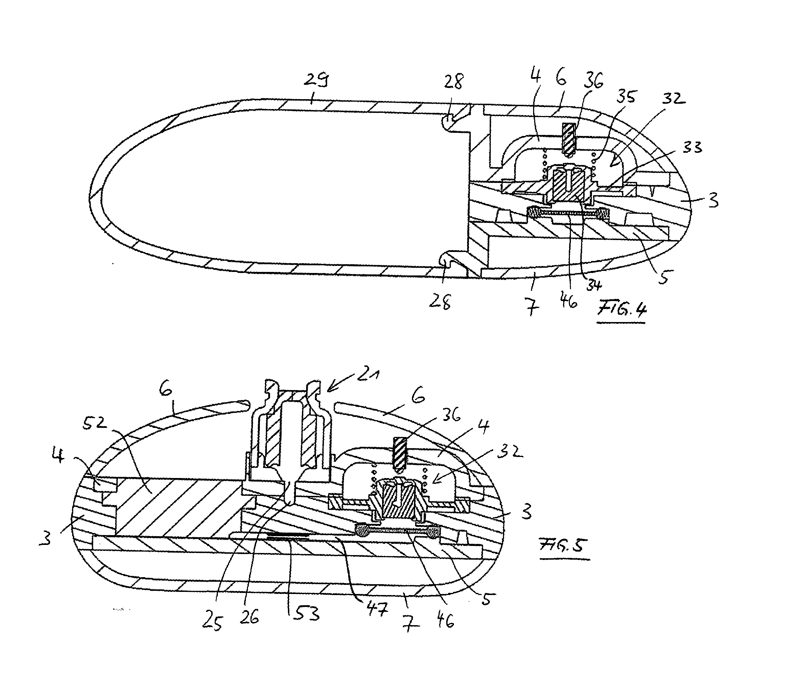

Durch das LuerLock-Ventil 31 und die Kanäle 25, 26 und 13 wird die Pumpe mit Flüssigkeit befüllt. Ausgehend von dem in Figur 2 veranschaulichten unbefüllten Zustand dehnt sich mit zunehmender Zugabe von Flüssigkeit der Ballon 16 in demjenigen Bereich, der nicht durch den Klemmring 20 geklemmt ist und nimmt die in Figur 3 veranschaulichte Endform bei vollständiger Füllung an. Der Raum, der von der Flüssigkeit eingenommen wird, ist dort mit der Bezugsziffer 27 bezeichnet. Den Figuren 2, 3 und 6 bis 12 ist zu entnehmen, dass sich der Ballon 16, ausgehend von seinem am Kern 11 anliegenden Ausgangszustand beim Befüllen mit Flüssigkeit seine Form sowohl in Längsrichtung des Kerns als auch in dessen Querrichtungen verändert, d. h. in einer ersten Querrichtung und einer senkrechten hierzu gesehen zweiten Querrichtung.

Das Oberteil 4 und das Unterteil 5 sind mit Rastvorsprüngen 28 versehen, die der Aufnahme einer im Querschnitt annähernd nierenförmig gestalteten Haube 29 dienen. Diese Haube besitzt, wie es anschaulich der Figur 9 zu entnehmen ist, eine Erstreckung in Breitenrichtung, die wesentlich größer ist als die in Höhenrichtung. Das Breiten-/Höhenverhältnis beträgt beispielsweise 2:1. Das Längen-/Höhenverhältnis der Haube 29 ist, wie es beispielsweise der Figur 2 zu entnehmen ist, ungefähr 2,5:1. Die Haube 29 ist vorzugsweise unlösbar mit dem Gehäuse 2 verclipst. Bei vollständig mit Flüssigkeit befülltem Ballon 16 nimmt dieser soviel wie möglich von dem Innenraum der Haube 29 ein.

Erreicht wird dies dadurch, dass, wie es der Darstellung der Figur 11 für den am Kern 11 anliegenden Ballon 16 zu entnehmen ist, der Ballon 16 in einer ersten Erstreckungsrichtung X senkrecht zur Längsachse des Kerns 11 relativ dicke Wandungsabschnitte 30 und in einer zweiten Erstreckungsrichtung Y senkrecht zur Längsachse des Kerns 11 und senkrecht zur ersten Erstreckungsrichtung X relativ dünne Wandungsabschnitte 31 aufweist. Demzufolge hat der Ballon 16 beim Einbringen von Flüssigkeit in dessen Raum 27 das Bestreben sich bevorzugt in Erstreckungsrichtung X auszudehnen, womit die ausgedehnte ovale Querschnittsform, wie sie in der Darstellung der Figur 9 veranschaulicht ist, sich ergibt. Insgesamt stellt sich die Pumpe 1 als flaches, günstig am Körper zu tragendes Funktionsteil dar, wobei der Ballon 16 in mit Flüssigkeit gefülltem Zustand gleichfalls eine flache Form einnimmt, die der äußeren Kontur der Pumpe 1 angepasst ist.

Die Kanäle 26 und 13 dienen nicht nur dem Zuführen der Flüssigkeit vom Luer-Lock-Ventil 21 in den Ballon 16, sondern auch dem Abführen der Flüssigkeit aus dem Inneren des Ballons 16 zum Patienten. So ist der Kanal 26 über die Zugangsstelle des Kanals 25 hinaus verlängert zu einem im Mittelteil 3 und im Oberteil 4 gelagerten Ventil 32 zum Begrenzen des von dem Ballon 16 ausgegebenen Flüssigkeits-Volumenstroms. Dieses Ventil 32 ist durch eine randseitig zwischen Mittelteil 3 und Oberteil 4 gehaltene elastische Ventilmembran 33, einen mit dieser zusammenwirkenden Ventilkern 34, eine sich an der Ventilmembran 33 und dem Oberteil 4 abstützende Druckfeder 35 und eine in einem Gewinde des Oberteils 4 gelagerte Stellschraube 36, die in Wirkverbindung mit der Ventilmembran 33 bringbar ist, gebildet.

Wie im Detail der Darstellung der Figur 13 zu entnehmen ist, mündet der Kanal 26 in einen radial verlaufenden Kanal 37 der Ventilmembran 33 und von dort in einen radialen Kanal 38 des Veritilkörpers 34, der in einen axialen Kanal 39 des Ventilkerns 34 mündet. Dieser Kanal 39 ist im Bereich seines, einem verstärkten Abschnitt 40 der Ventilmembran 33 zugewandten Endes offen ausgebildet. Auf der dem Kanal 39 abgewandten Seite des Abschnitts 40, dem die Funktion eines Verschlusselements zukommt, ist ein Anschlag, der als Stellschraube 36 ausgebildet ist, angeordnet. Grundsätzlich könnte dieser Anschlag auch stationär sein. Zwischen den Vorsprüngen 41 der Ventilmembran 33 ist der Ventilkern 34 axial unverschieblich bezüglich der Ventilmembran 33 gehalten und im Übrigen auch bezüglich dieser nicht drehbar.

Das Ventil 32 dient dem Sperren des Volumenstromes bei Anliegen eines zu hohen Drucks. Im Ventil sind zwei getrennte Kammern 42 und 43 gebildet, die miteinander über einen den Ventilkern 34 durchsetzenden, parallel zum Kanal 40 angeordneten Kanal 44 verbunden sind. Dabei dient die Kammer 42, welche in Strömungsrichtung zum Zugang, demnach zum Kanal 26 liegt, als Sperrkammer. Die Kammer 43 liegt in Strömungsrichtung zum Abgang 45. Zum Filtern der durch das Ventil 32 ausgegebenen Flüssigkeit ist ein Filter 46 vorgesehen, der randseitig zwischen dem Mittelteil 3 und dem Unterteil 5 geklemmt ist. Ausgehend von der Kammer 43 und dem Abgang 45 gelangt die Flüssigkeit zu einem mit dem Abgang 45 in Fließverbindung stehenden Kanal 47 (Figur 5) und von dort zu einem zwischen dem Mittelteil 3 und dem Unterteil 5 gehaltenen LuerLock-Anschluss 48. Mit diesem ist ein Luer-Lock-Verbinder 49 verbindbar, der mit einem Schlauch 50, der zum Patienten führt, versehen ist.

Wie der Darstellung der Figur 5 zu entnehmen ist, ist in den Kanal 47 eine Glaskapillare 53 eingesetzt. Diese stellt einen Durchflussbegrenzer dar, der in der Lage ist, den Volumenstrom, der durch den Kanal 47 aus der Pumpe austritt, zu begrenzen, weil der Durchflussbegrenzer einen kleineren Querschnitt aufweist, als der im Zugang liegende Kanal 37. Durch die Wahl verschiedener Durchflussbegrenzer ist die Einstellung verschiedener konstanter Flowraten möglich, solange der Eingangs des Eintritts anliegende Druck einen bestimmten Wert nicht unterschreitet. Grundsätzlich ist vorgesehen, dass der Durchflussquerschnitt des Zugangs größer ist als der Durchflussquerschnitt des Abgangs. Selbstverständlich kann der Durchflussbegrenzer anders ausgeführt sein, als in Art einer Glaskapillare. Es ist durchaus denkbar, hinter dem Ventil im Abgang der Pumpe, beispielsweise einen Mäander-Chip, der den Durchfluss begrenzt, vorzusehen.

Auf Grund der angegebenen Durchmesser der Kanäle, die den Raum 27 des Ballons mit dem Ventil 32 verbinden und des Durchmessers der hinter dem Ventil 32 angeordneten Kanäle mit dem Durchflussbegrenzer 53, ist der Widerstand, den der Kanal 47 mit dem Durchflussbegrenzer 53 dem Auströmen der Flüssigkeit aus dem Gehäuse 2 entgegensetzt, größer als der Widerstand, der der Flüssigkeit beim Einströmen in das Ventil 32 entgegen gesetzt wird.

In einer Ausgangssituation befindet sich die Ventilmembran 33 in der in Figur 13 gezeigten Position, in der die Ventilmembran 33, ohne dass es einer Einwirkung der Druckfeder 35 bedürfte, weitgehend am Mittelteil 3 anliegt. Auf Grund der Positionierung des Ventilkerns 34 bezüglich des Abschnitts 40 der Ventilmembran 33 ist ein geringer Spalt zwischen dem Abschnitt 40 und einem umlaufenden, somit ringförmigen Vorsprung 54 des Ventilkerns 34 gegeben. Dieser Vorsprung 54 umschließt den Kanal 43. Demzufolge strömt Flüssigkeit über den Kanal 13 des Kerns 11 und den anschließenden, gehäuseseitigen Kanal 26 in den Kanal 37 der Ventilmembran 33 und von dort in die Kanäle 38 und 39 des Ventilkerns 34. Vom Kanal 39 des Ventilkerns 34 strömt die Flüssigkeit durch den zwischen dem Vorsprung 54 und dem Abschnitt 40 der Ventilmembran 33 gebildeten Spalt in die dort befindliche Kammer 42, und von der Kammer 42 über den Kanal 44 zwischen Ventilmembran 33 und Ventilkern 34 zur Kammer 43, passiert den Filter 46 und gelangt über den Abgang 45 zum Kanal 47 mit dem Durchflussbegrenzer 53. Stellt sich im Einlass, somit auch im Kanal 39, ein höherer Flüssigkeitsdruck ein, ohne dass in Folge des Durchflussbegrenzers 43 ein größerer Volumenstrom aus der Pumpe austreten kann, führt dies dazu, dass die Ventilmembran 33, die in dem Randbereich zwischen dem Mittelteil 3 und dem Oberteil 4 geklemmt ist, sich im zentralen Bereich in Richtung der die Anschlagfunktion aufweisenden Stellschraube 36 verformt, und zwar entgegen der Kraft der Druckfeder 35. Gelangt die Ventilmembran 34 mit ihrem Abschnitt 40 gegen den Vorsprung 55 der Stellschraube 36, der dem Abschnitt 40 zugewandt ist, kontaktiert der Abschnitt 40 dort die Stellschraube 36, womit, da sich die Ventilmembran 33 nicht weiter nach oben in Richtung der Oberschale bewegen kann, der Abschnitt 40 gegen den Vorsprung 54 des Ventilkerns 34 gedrückt wird und damit den Durchfluss durch den Kanal 39 verschließt. Beim Abfließen der Flüssigkeit durch den Durchflussbegrenzer 53 baut sich der Druck in der Kammer 43 ab, sodass die Membran, durchaus auf Grund deren eigenen Elastizität, wieder in Richtung ihrer Ausgangsstellung gemäß Figur 13 zurückbewegt, damit der Abschnitt 40 außer Kontakt mit der Stellschraube 36 gelangt und der Durchflussspalt zwischen dem Vorsprung 54 und dem Abschnitt 40 wieder freigegeben wird. In Abhängigkeit vom Druck, der in dem Ballon 16 herrscht, kann dieser Zustand erst bei Erreichen der Ausgangsposition der Ventilmembran 33, wie sie in Figur 13 dargestellt ist, oder durchaus schon früher, somit bei noch ausgelenkter Ventilmembran 33, eintreten. Die Stellschraube 36 dient der Veränderung des Öffnungs-Schließ-Verhaltens des Ventils 32. Je weiter die Schraube den Stellweg der Ventilmembran freigibt, desto größer ist der Sekundärdruck im Ventil. Grundsätzlich ist es nicht erforderlich, die Druckfeder 35 vorzusehen. Sie ist dann von Vorteil, wenn größere Drücke mit der Pumpe 1 beherrscht werden sollen und demzufolge das elastische Rückstellverhalten der Ventilmembran 33 nicht ausreicht, um sie in die Ausgangsstellung gemäß Figur 13 zu überführen.

Mit dem Ventil 32 wird somit der Flüssigkeits-Volumenstrom in Abhängigkeit vom anstehenden Druck im Ballon 16 begrenzt und über den Durchflussbegrenzer 53 der Flüssigkeits-Volumenstrom weitgehend konstant gehalten. Grundsätzlich könnte die Flüssigkeitspumpe so modifiziert werden, dass nur eine Einrichtung zum weitgehenden Konstanthalten des vom elastischen Element ausgegebenen Flüssigkeits-Volumenstroms oder eine Einrichtung zum Begrenzen des vom elastischen Element ausgegebenen Flüssigkeits-Volumenstroms vorgesehen ist.

Vor dem Benutzen der mechanisch betriebenen Flüssigkeitspumpe wird Flüssigkeit durch das Luer-Lock-Ventil 21 zugeführt, womit die Flüssigkeit in den Ballon 16 gelangt und der Füllstand des Ballons sich durch die durchsichtige Haube 29 an Hand der in Querrichtung der Haube angebrachten Markierungen 51 ablesen lässt, die eine Referenz für die Querausdehnung des Ballons in Abhängigkeit von dessen Befüllung sind. Nach dem Befüllen der Pumpe 1 und dem Anschließen der Pumpe an den Patienten über den Schlauch 50, wird, unter elastischer Vorspannung des aufgeweiteten Ballons 16, Flüssigkeit über das Ventil 32 aus der Pumpe ausgegeben, und zwar solange, bis der Ballon restlos entleert ist und an dem Kern 11 anliegt.

Durch die besonders einfache Gestaltung der beschriebenen Flüssigkeitspumpe bietet diese vielfältige Möglichkeiten der Verwendung bzw. Anwendung. Der Anwender kann überall, sofort, ohne längere Anlaufzeiten die Pumpe einsetzen. Sie kann vom Anwender tragbar, wie auch statisch benutzt werden, und zwar in allen normalen Lebensbereichen außerhalb, wie innerhalb der Medizin. Die Pumpe ist sterilisiert einsetzbar und gewährleistet einen minimalen Bedienungs/Handlingaufwand. Die Pumpe ist auf Grund der einfachen Konstruktion der wenigen Bauteile kostengünstig herstellbar. Dies ist Vorraussetzung dafür, dass sie insbesondere in ambulanten und finanzschwachen Märkten eingesetzt werden kann. Das geringe Gewicht der Pumpe ermöglicht den Einsatz im Unfall-, Notarzt-, Lazarett- sowie Katastrophenbereich. Die Funktionselemente der Pumpe sind einzeln oder insgesamt austauschbar. Die Pumpe ist für kurze oder lange Förderzeiten geeignet, beispielsweise bei einem Ballon mit einem Fassungsvermögen von 25 ml für eine Durchflussrate von 2,5 ml pro Stunde, somit einer Laufzeit von 10 Stunden. Selbstverständlich können andere Ballons Verwendung finden, die andere Volumina aufweisen, z. B. 10 ml, 50 ml, 100 ml oder 150 ml. Die Laufzeit kann durchaus wesentlich länger sein, beispielsweise bis 24 Stunden. Obwohl Flowraten von > 1000 ml pro Stunde durchaus denkbar sind, wird als bevorzugtes Anwendungsgebiet eine Flowrate von 0,5 bis 10 ml pro Stunde angesehen.

Gemäß dem Ausführungsbeispiel wird eine spritzgusstechnisch hergestellter Ballon beschrieben, der als Sammelbehälter für die Medikamentenlösung und Druckreservoir dient. Der Ballon weist eine definierte Kontur im Querschnitt und in der Ausdehnung, zum Füllen flacher Gehäuseräume und zur Vermeidung von Druckspitzen auf. Er steht radial und/oder axial unter Vorspannung über einen ein- oder mehrteiligen Kern, zur Erhöhung der Rückstellkräfte. Der Ballon wird einseitig über den Kern ortsfest mittels eines Klemmrings formschlüssig, luftdicht abgeschlossen. Der Ballon ist frei beweglich in axialer und radialer Richtung während der Befüllung und Entleerung, dabei elastisch verformbar und kann sich reibungsfrei innerhalb der Haube bewegen.

Die Pumpe 1 kann zusätzlich mit einem Bolusreservoir versehen sein. In der Figur 1 ist eine solche Bolus-Vorsehung mit der Bezugsziffer 52 veranschaulicht. Bei Bedarf kann die Pumpe insofern umgerüstet werden.

Die Figur 14 veranschaulicht in einem Schaubild das Wirkprinzip der zuvor beschriebenen mechanisch betriebenen Flüssigkeitspumpe mit Angabe der physikalischen Größen. Mit strichpunktierter Linie ist die Kontur der Pumpe verdeutlicht.

Die Figur 15 zeigt, betreffend die Bolusschuss-Implementierung, hinter der Kapillare K, somit hinter der Einrichtung zum weitgehenden Konstanthalten des von dem elastischen Element BK ausgegebenen Flüssigkeits-Volumenstroms, das Bolusreservoir BR. Die zur Kapillare K führende Leitung ist mit ZF, die vom Bolusreservoir wegführende Leitung mit AF bezeichnet. Figur 16 zeigt im Unterschied zu der in Figur 15 gezeigten Reihenschaltung von Kapillare K und Bolusreservoir BR deren Parallelschaltung.

Figur 17 veranschaulicht die Gestaltung des Bolusreservoirs statt der in der Figur 1 mit der Bezugsziffer 52 bezeichneten Bolus-Vorsehung: Bei einer Reihenschaltung im Sinne der Darstellung in Figur 15 mündet der Kanal 47 stromabwärts der Kapillare 53 (Figur 5) in eine als Vertiefung im Unterteil 5 ausgebildete Boluskammer 56, die durch eine elastische Membran 57, die zwischen dem Mittelteil 3 und dem Unterteil 5 in deren Randbereich geklemmt ist, gegenüber einer Ausnehmung 58 im Mittelteil 3 abgedichtet ist. Die Ausnehmung 58 dient der Aufnahme eines Stößels 59, der in Richtung des Pfeiles F entgegen der Rückstellkraft eines elastischen Siliconrings 60 gegen die Membran 57 verschiebbar ist, so dass er die Membran 57 in die Boluskammer 56 bewegt, womit schlagartig Flüssigkeit aus der Boluskammer 56 zum Auslass der Pumpe, somit zum LuerLock-Anschluss 48 austritt. Sobald kein Druck mehr von außen auf den Stößel 59 ausgeübt wird, gelangt dieser unter der Kraft des elastischen Siliconrings 60 wieder in seine in Figur 17 gezeigte Ausgangslage, in der das Volumen der Boluskammer 56 wieder vergrößert ist und der Stößel 59 mit einem Vorsprung am Oberteil 4 anliegt und im übrigen einen Durchgang 61 im Oberteil 4 durchsetzt. Statt des elastischen Siliconrings kann beispielsweise auch eine Druckfeder vorgesehen sein, die den Stößel 59 umschließt und die Aufgabe hat, diesen in seine die Boluskammer 56 freigebende Stellung zu überführen, wie sie in Figur 17 verdeutlicht ist.

Mittels der Bolusschuss-Einrichtung ist somit eine diskrete Verabreichung dosierter Einzelgaben von Medikamentenlösungen möglich.

## Patentansprüche

1. Mechanisch betriebene Flüssigkeitspumpe (1), insbesondere für medizinische oder ernährungsphysiologische Flüssigkeiten, sowie Flüssigkeiten im biologischen und Laborbereich, mit folgenden, innerhalb eines Gehäuses (2) der Flüssigkeitspumpe (1) gelagerten Funktionsteilen:
- einem verschließbaren Zugang (21) zum Zuführen der Flüssigkeit,
- einem aufweitbaren elastischen Element (16) zum Speichern und Abgeben der über den verschließbaren Zugang (21) zugeführten Flüssigkeit,
- einer Einrichtung (53, 32) zum weitgehenden Konstanthalten und/oder Begrenzen des von dem elastischen Element (16) ausgegebenen Flüssigkeits-Volumenstroms,
- einem aus dem Gehäuse (2) führenden Auslass (48).

2. Pumpe nach Anspruch 1, **dadurch gekennzeichnet, dass** die Funktionsteile kalibriert sind.

3. Pumpe nach Anspruch 1 oder 2, **dadurch gekennzeichnet, dass** das Gehäuse (2), zumindest in einem Bereich, der den Zugang (21), die Einrichtung (53, 32) zum weitgehenden Konstanthalten und/oder Begrenzen des von dem elastischen Element (16) ausgegebenen Flüssigkeits-Volumenstroms und den Auslass (48) aufweist, insbesondere das Gehäuse (2) insgesamt, nicht zerstörungsfrei demontierbar ist.

4. Pumpe nach einem der Ansprüche 1 bis 3, **dadurch gekennzeichnet, dass** der Zugang (21) und/oder der Abgang (48) als LuerLock-Ventil (21) oder LuerLock-Anschluss (48) ausgebildet ist.

5. Pumpe nach einem der Ansprüche 1 bis 4, **dadurch gekennzeichnet, dass** das aufweitbare elastische Element (16) als Ballon (16) ausgebildet ist, der mit einer Öffnung (17a) versehen ist, wobei der Ballon (16) abgedichtet in einem im Gehäuse (2) gelagerten Kern (11) gelagert ist, und den Kern (11) mindestens einen Kanal (13, 14) für den Zugang und die Abgabe der Flüssigkeit in den Ballon (16) bzw. aus dem Ballon (16) durchsetzt.

6. Pumpe nach einem der Ansprüche 1 bis 5, **dadurch gekennzeichnet, dass** die Einrichtung (32) zum Begrenzen des vom elastischen Element (16) ausgegebenen Flüssigkeits-Volumenstroms als Ventil (32), insbesondere als Druckregelventil ausgebildet ist.

7. Pumpe nach einem der Ansprüche 1 bis 6, **dadurch gekennzeichnet, dass** die Einrichtung (53) zum weitgehenden Konstanthalten des vom elastischen Element (16) ausgegebenen Flüssigkeits-Volumenstroms als Durchflussbegrenzer (53), insbesondere als Glaskapillare oder Mäander-Chip ausgebildet ist.

8. Pumpe nach einem der Ansprüche 1 bis 7, **dadurch gekennzeichnet, dass** innerhalb des Gehäuses (2), insbesondere stromaufwärts der Einrichtung (53) zum weitgehenden Konstanthalten des von dem elastischen Element (16) ausgegebenen Flüssigkeits-Volumenstroms, ein Filterelement (46) für die Flüssigkeit angeordnet ist.

9. Pumpe nach einem der Ansprüche 1 bis 8, **dadurch gekennzeichnet, dass** das Gehäuse (2) ein Mittelteil (3), ein Oberteil (4) und ein Unterteil (5) aufweist.

10. Pumpe nach einem der Ansprüche 1 bis 9, **dadurch gekennzeichnet, dass** das Gehäuse (2) eine mit dem Oberteil (4) und dem Unterteil (3) verbundene, insbesondere unlösbar verclipsbare Haube (29) zur Anordnung des Ballons (16) und des Kerns (11) innerhalb der Haube (29) aufweist.

11. Pumpe nach Anspruch 9 oder 10, **dadurch gekennzeichnet, dass** im Mittelteil (3) und im Oberteil (4) oder Unterteil (5), zwischen dem Mittelteil (2) und dem Oberteil (4) oder dem Unterteil (5), der Kern (11) für den Ballon (16) und/oder der Auslass (48) und/oder das Filterelement (46) und/oder die Einrichtung (32) zum Begrenzen des vom elastischen Element (16) ausgegebenen Flüssigkeits-Volumenstroms gelagert ist/sind.

12. Pumpe nach einem der Ansprüche 9 bis 11, **dadurch gekennzeichnet, dass** im Mittelteil (3) die Einrichtung (53) zum Begrenzen des vom elastischen Element (16) ausgegebenen Flüssigkeits-Volumenstroms und/oder eine Boluseinrichtung (56, 57, 59, 60) gelagert ist.

13. Pumpe nach einem der Ansprüche 9 bis 12, **dadurch gekennzeichnet, dass** im Oberteil (4) oder Unterteil (5) der Zugang (21) gelagert ist.

14. Pumpe nach einem der Ansprüche 9 bis 13, **dadurch gekennzeichnet, dass** ein Gehäuseoberteil (6) zum Abdecken des Oberteils (4) und ein Gehäuseunterteil (7) zum Abdecken des Unterteils (5) vorgesehen sind.

15. Pumpe nach einem der Ansprüche 9 bis 14, **dadurch gekennzeichnet, dass** die Teile (3, 4, 5, 6, 7, 29) des Gehäuses (2) aus Kunststoff bestehen und Teile des Gehäuses (2) durch Laserschweißen miteinander verbunden sind.

16. Pumpe nach einem der Ansprüche 9 bis 15, **dadurch gekennzeichnet, dass** das Mittelteil (3) eingeprägte Kanäle aufweist.

17. Pumpe nach einem der Ansprüche 1 bis 16, **dadurch gekennzeichnet, dass** das Gehäuse (2) flach ausgebildet ist.
